# EUROPÄISCHE PATENTSCHRIFT

(11) **EP 0 753 542 B1**
(45) Veröffentlichungstag und Bekanntmachung des Hinweises auf die Patenterteilung: **09.02.2000**
(21) Anmeldenummer: 96110594.7
(22) Anmeldetag: 01.07.1996
(51) Int. Cl.: C09B 35/60, C09B 35/025, C09B 43/16, C09D 11/00, C07D 251/70

(54) **Polyazofarbstoffe**
Polyazo dyestuffs
Colorants polyazoiques

(30) Priorität: 14.07.1995 DE 19525613; 17.08.1995 DE 19530202
(43) Veröffentlichungstag der Anmeldung: 15.01.1997
(73) Patentinhaber: BAYER AG, 51368 Leverkusen (DE)
(72) Erfinder: Stöhr, Frank-Michael, Dr., 51519 Odenthal (DE); Wild, Peter, Dr., 51519 Odenthal (DE)

(56) Entgegenhaltungen:
- EP-A- 0 190 586
- EP-A- 0 645 434
- FR-A- 2 378 069
- FR-A- 2 424 305
- GB-A- 2 082 615
- GB-A- 2 266 893
- US-A- 2 258 977

## Beschreibung

Die Erfindung betrifft neue Polyazofarbstoffe, ein Verfahren zu ihrer Herstellung sowie ihre Verwendung zum Färben und Bedrucken von hydroxyl- und/oder amidgruppenhaltigen Substraten, insbesondere zum Bedrucken von Papier nach der Ink-Jet-Methode.

In GB-A-2 082 615 und FR-A-2 424 305 werden Azogruppen-haltige Verbindungen offenbart, die an den endständigen Phenylringen Aminotriazinylreste tragen, welche ihrerseits mit Alkylendiaminen substituiert sind.

In GR-A-2 266 893 werden Disazofarbstoffe offenbart, an deren endständigen Naphthalinringen Aminotriazinylreste gebunden sind.

EP-A-645 434 offenbart Trisazofarbstoffe, die mit einem Aminotriazinylrest an einem terminalen Phenylring substituiert sind.

EP-A-190 586 offenbart Disazoverbindungen, die aus einer zweifach ankuppelbaren Kupplungskomponente und zwei endständigen Diazokomponenten aufgebaut sind.

Aus FR-A-2 378 069 sind Monoazofarbstoffe bekannt, die an ihrer phenylischen Kupplungskomponente einen Aminotriazinylrest tragen, der seinerseits mit aliphatischen Aminresten substituiert sein kann.

Aus US-A-2258 977 sind Monoazofarbstoffe bekannt, die einen Aminotriazinylrest tragen.

Polyazofarbstoffe sind bereits aus JP-A-1 197 580 und JP-A-1 153 775 bekannt. Diese weisen jedoch noch einige anwendungstechnische Nachteile auf.

Die vorliegende Erfindung betrifft neue Verbindungen der Formel (I) 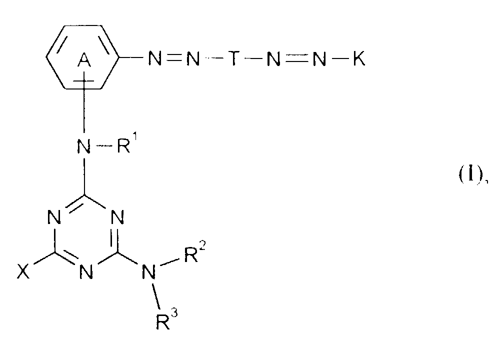 worin
- T: für den Rest einer Tetrazokomponente steht,
- R¹: Wasserstoff oder unsubstituiertes oder substituiertes C₁-C₄-Alkyl bedeutet,
- R² und R³: zusammen mit dem N-Atom, an das sie gebunden sind, einen gesättigten oder ungesättigten 3- bis 8-, insbesondere 5- oder 6-gliedrigen Ring bilden, der entweder keine weiteren oder 1 bis 2 weitere Heteroatome aus der Gruppe O, S, SO, SO₂, NR, mit R = H oder unsubstituiertes oder substituiertes C₁-C₆-Alkyl, enthält und unsubstituiert oder durch C₁-C₄-Alkyl, C₁-C₄-Alkyl, C₁-C₄-Hydroxyalkyl oder C₁-C₄-Aminoalkyl substituiert ist,
- X: für NR²R³ steht, wobei R² und R³ die obengenannte Bedeutung besitzen, und der Ring A gegebenenfalls weitere Substituenten trägt,
- K: einen Rest der Formel (II) 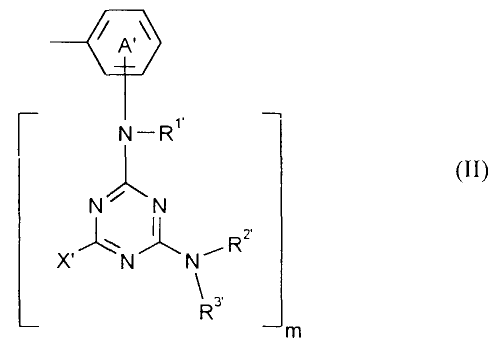
bedeutet, worin
R^{1'}, R^{2'}, R^{3'}, X' und A' die Bedeutungen der entsprechenden Substituenten R¹, R², R³, X und A annehmen können, jedoch unabhängig davon sind und m für 1 steht.

Als Substituenten von Alkylgruppen sind OH, C₁-C₄-Alkoxy, Halogen, wie Cl, F oder Br, COOH, SO₃H, OSO₃H und/oder NR⁴R⁵ bevorzugt, wobei R⁴ und R⁵ unabhängig voneinander für Wasserstoff oder unsubstituiertes oder durch wasserlöslichmachende Gruppen substituiertes Alkyl, insbesondere C₁-C₄-Alkyl, stehen.

Unter wasserlöslichmachende Gruppen sind insbesondere SO₃H, OSO₃H, COOH, zu verstehen.

Als weitere Substituenten für den Ring A sind vorzugsweise zu nennen: C₁-C₄-Alkyl, C₁-C₄-Alkoxy, Halogen, OH, -S-C₁-C₄-Alkyl, NR⁴R⁵, worin R⁴ und R⁵ die obengenannte Bedeutung haben, SO₃H, COOH, -NR¹COC₁-C₄-Alkyl, -NR¹COC₆-C₁₀-Aryl, -NR¹-SO₂-C₁-C₄-Alkyl, -NR¹-SO₂-C₆-C₁₀-Aryl, -NR¹CONH₂, -NR¹-COCH₂OH; NR¹COOC₁-C₄-Alkyl, worin R¹ die oben angegebene Bedeutung hat und NR²R³, worin NR²R³ die obige Bedeutung für einen heterocyclischen Ring besitzt.

Ganz besonders bevorzugt sind Polyazofarbstoffe, worin A' = A, R^{1'} = R¹, R^{2'} = R², R^{3'} = R³, und X' = X.

Bevorzugte Reste der Tetrazokomponente T entsprechen der Formel (III) 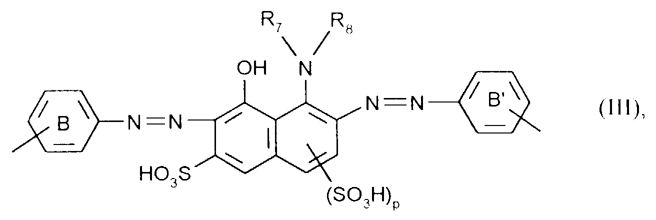 worin
- R⁷ und R⁸: unabhängig voneinander Wasserstoff oder unsubstituiertes oder substituiertes C₁-C₄-Alkyl bedeuten, wobei als mögliche Substituenten die bereits für Alkylreste oben genannten bevorzugt sind,
die Ringe B und B' unabhängig voneinander entweder keine weiteren oder weitere Substituenten tragen, wobei als Substituenten C₁-C₄-Alkyl, C₁-C₄-Alkoxy, Halogen, SO₃H und/oder COOH bevorzugt sind und p 0 oder 1 bedeutet.

Ebenfalls bevorzugte Reste der Tetrazokomponente T entsprechen der Formel (IV) 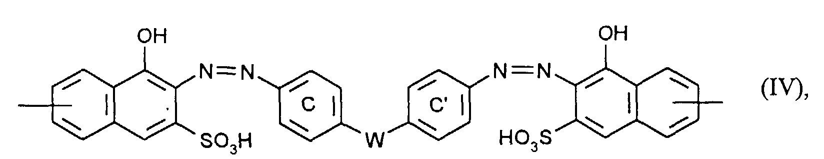 worin
die Ringe C und C' unabhängig voneinander entweder keine weiteren oder weitere Substituenten tragen, wobei als Substituenten C₁-C₄-Alkyl, C₁-C₄-Alkoxy, Halogen, SO₃H und/oder COOH bevorzugt sind und
W für ein Brückenglied steht.

Als bevorzugte Brückenglieder für W seien genannt: 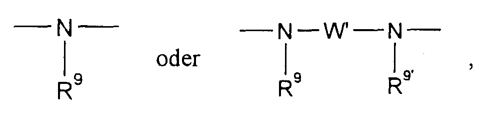 worin W' für einen Rest der Formel 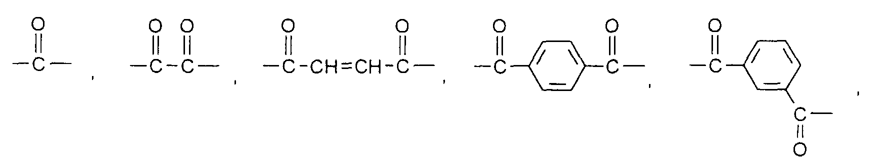 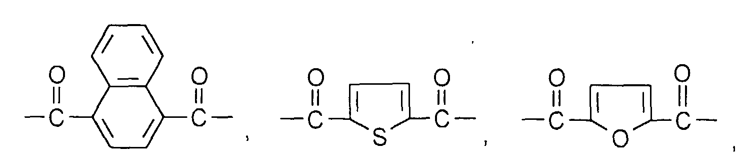 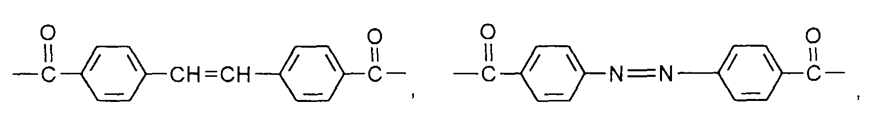 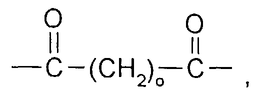 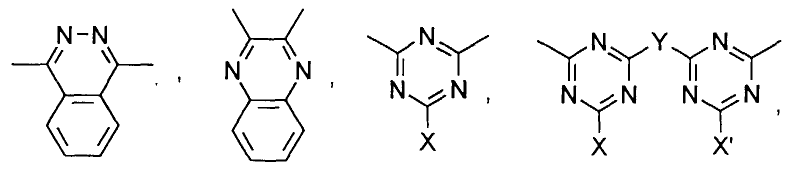 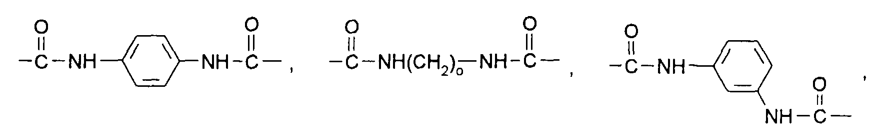 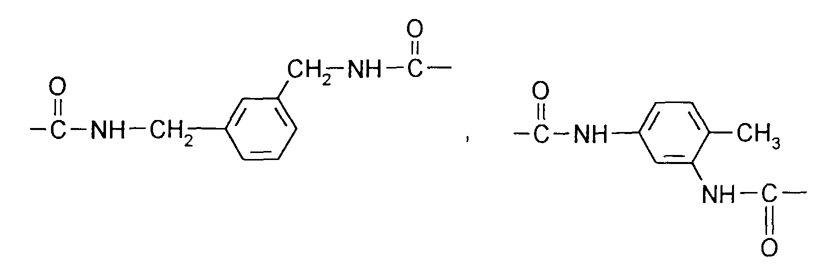 steht,
worin
- R⁹ und R^{9'}: unabhängig voneinander für Wasserstoff oder gegebenenfalls substituiertes C₁-C₄-Alkyl stehen,
- Y: für ein Brückenglied steht,
- o: für eine Zahl von 1 bis 6 steht und
- X und X': unabhängig voneinander die oben angegebene Bedeutung haben.

Als Beispiele für Y seien genannt 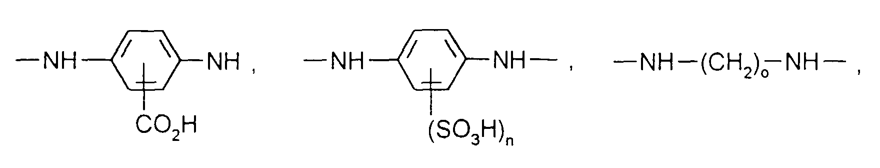 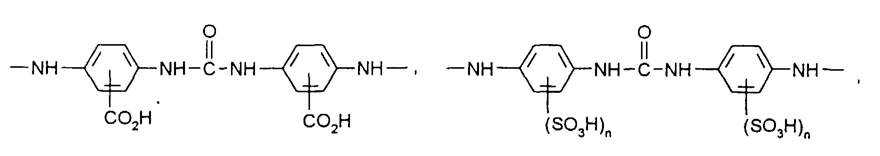 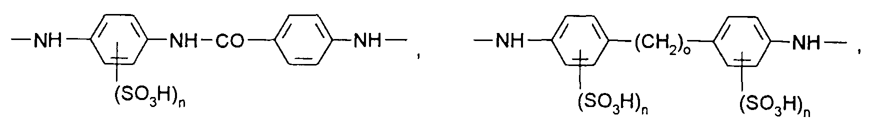 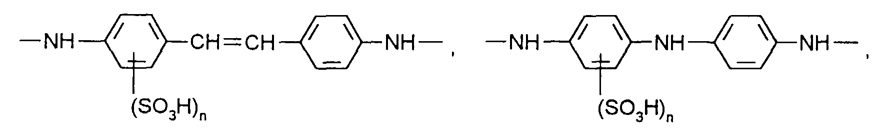 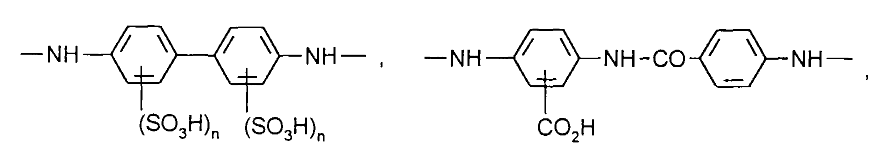 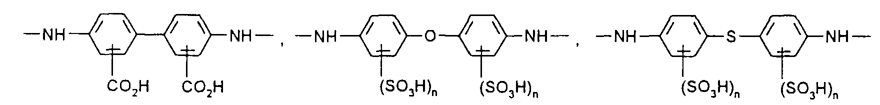 worin
- o: für 1 bis 6 steht und
- n: für 0 oder 1 steht.

Als bevorzugte Reste in der Bedeutung von 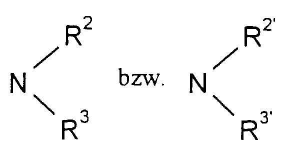 sind die Reste der nachfolgenden Formeln zu nennen 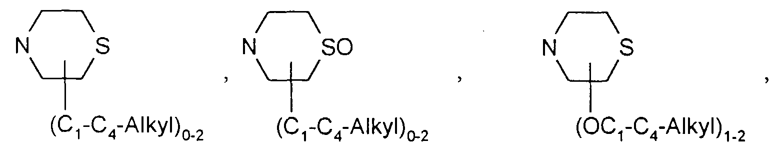 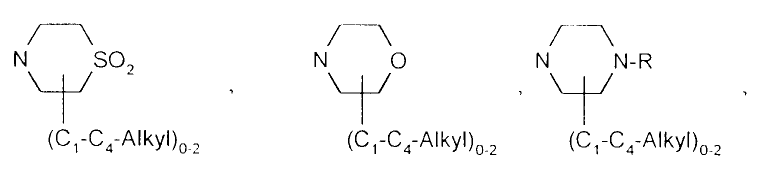 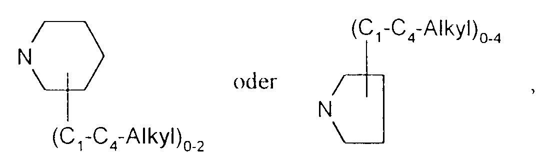 worin
- R: die oben angegebene breiteste Bedeutung hat.

Besonders bevorzugte Reste in der Bedeutung von 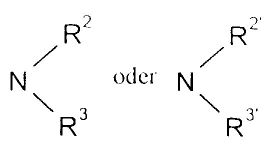 sind

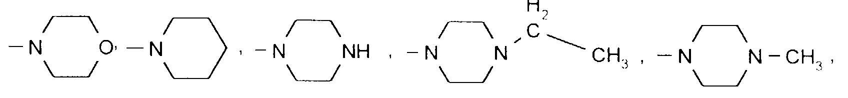

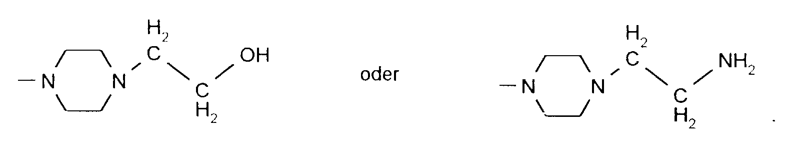

Ganz besonders bevorzugt sind Farbstoffe der Formel (I), die der Formel (V) entsprechen 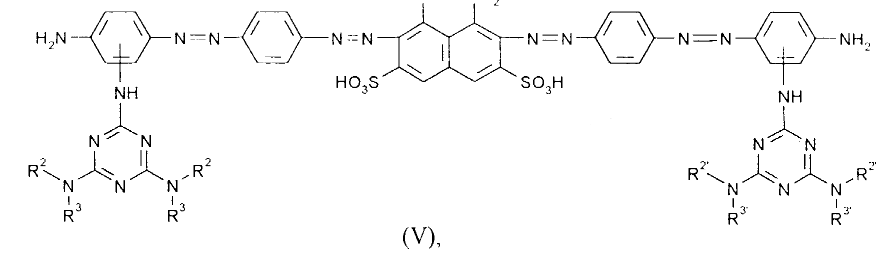 worin
- R² und R³: sowie R^{2'} und R^{3'} jeweils unabhängig voneinander mit dem jeweiligen N-Atom, an das sie gebunden sind, einen gesättigten oder ungesättigten 3-bis 8-, insbesondere 5- oder 6-gliedrigen Ring bilden, der keine weiteren oder 1 bis 2 weitere Heteroatome aus der Gruppe O, S, SO₂, SO oder NR mit R = H oder unsubstituiertes oder substituiertes C₄-C₆-Alkyl enthält und unsubstituiert oder durch C₁-C₄-Alkyl, C₁-C₄-Alkoxy, C₁-C₄-Hydroxyalkyl oder C₁-C₄-Aminoalkyl substituiert ist.

Ein ganz besonders bevorzugter Farbstoff der Formel (V) entspricht der Formel

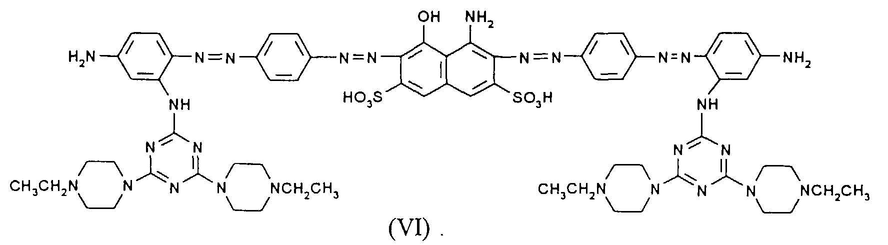

Die Erfindung betrifft weiterhin ein Verfahren zur Herstellung von Farbstoffen der Formel (I), das gekennzeichnet ist durch Tetrazotierung der Diamine der Formel (VII)

H₂N - T - NH₂ (VII)

und Kupplung auf die Kupplungskomponenten der Formel 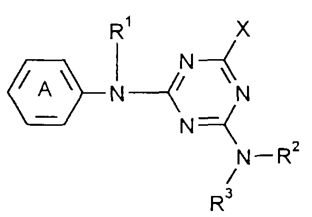 und H - K,
worin
R¹, R², R³, A, X, T und K die obige breiteste Bedeutung besitzen.

Die Kupplung kann gleichzeitig oder aufeinanderfolgend mit Kupplungskomponenten der Formel 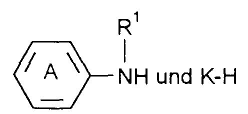 durchgeführt werden
und deren Umsetzungsprodukt dann mit einer Reaktivkomponente der Formel 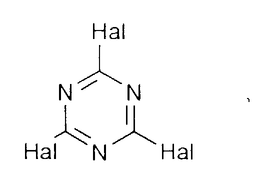 worin
Hal für Cl, F oder Br, insbesondere für Cl steht, und anschließend mit HX und 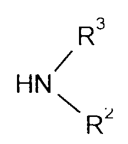 in beliebiger Reihenfolge umgesetzt werden.

Die Kupplung kann ebenfalls mit einer Kupplungskomponente der Formel 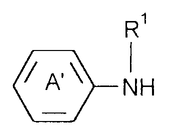 erfolgen
und anschließend mit einer Reaktivkomponente der Formel 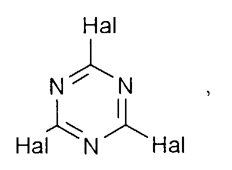 worin
Hal für Cl, Br oder F steht und
danach mit HX und HNR²R³ in beliebiger Reihenfolge umgesetzt werden.

Die Kupplungsreaktion läuft vorzugsweise in Gegenwart von säurebindenden Mitteln ab.

Geeignete säurebindende Mittel sind beispielsweise Alkalien oder Erdalkalien wie LiOH, NaOH, KOH, Ca(OH)₂, Na₂CO₃, NaHCO₃, Li₂CO₃.

Die Kupplungskomponente der Formel 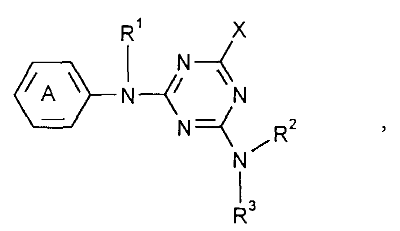 worin die Substituenten die oben angegebene Bedeutung besitzen, kann durch Kondensation von Verbindungen der Formel 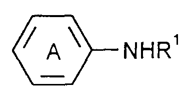 mit Cyanurhalogeniden, insbesondere Cyanurchlorid, und Verbindungen der Formel H-X und HNR²R³ in beliebiger Reihenfolge hergestellt werden. Die Kupplungskomponente H-K, die der Formel 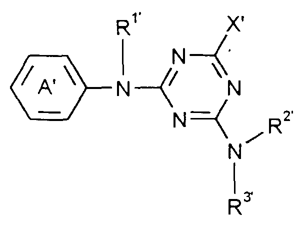 entspricht, kann analog hergestellt werden.

Für den Fall, daß der Ring A bzw. A' einen NO₂- oder NHCOAlkyl-Substituenten trägt, wird vor der Kupplung sinnvollerweise reduziert bzw. verseift.

In einer bevorzugten Ausführungssform des erfindungsgemäßen Verfahrens wird nur eine Kupplungskomponente verwendet, womit die Farbstoffe der Formel (I) zwei identische Enden besitzen. Diese Kupplungskomponente hat die Formel 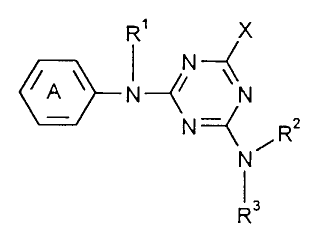 worin R¹ bis R³, X und A die oben angegebenen Bedeutungen besitzen.

Aber auch Verbindungen der Formel (I) mit unterschiedlichen Enden können nach dem erfindungsgemäßen Verfahren hergestellt werden, beispielsweise durch Umsetzung mit verschiedenen Kupplungskomponenten der sogenannten Mischkupplung.

Die Verbindungen der Formel (I) werden vorzugsweise als Farbstoffe zum Färben und Bedrucken von hydroxyl- und/oder amidgruppenhaltigen Substraten eingesetzt und färben cellulosehaltige Materialien in schwarzen Tönen, insbesondere Papier, Baumwolle und Viskose sowie Leder mit guten Naß- und Lichtechtheiten.

Die Farbstoffe können nach allen in der Papier- und Textilindustrie für substantive Farbstoffe gebräuchlichen Verfahren verwendet werden, insbesondere in der Massen- wie in der Oberflächenfärbung von Papier für geleimte oder ungeleimte Sorten, ausgehend von gebleichten oder ungebleichten Zellstoff verschiedener Provenienz wie Nadel- oder Laubholz-sulfit- und/oder -sulfat-Zellstoff. Sie können auch in der Garn- und Stückfärberei von Baumwolle, Viskose und Leinen nach dem Ausziehverfahren aus langer Flotte oder in Kontinueverfahren angewandt werden.

Weiterer Gegenstand der Erfindung ist ein Verfahren zum Färben von cellulosehaltigen Materialien mit den Farbstoffen der Formel (I).

Die erfindungsgemäßen Verbindungen der Formel (I) können als feste oder flüssige Farbstoffpräparationen eingesetzt werden. Sie werden vorzugsweise in Form von wäßrigen Präparationen, insbesondere von Lösungen eingesetzt. Diese wäßrigen Farbstoffpräparationen enthalten im allgemeinen einen oder mehrere Farbstoffe der Formel (I), gegebenenfalls geeignete organische Lösungsmittel, worunter auch hydrotrope Verbindungen zählen können sowie weitere Hilfsmittel und/oder Stabilisatoren. Es ist vorteilhaft, die wäßrigen Farbstofflösungen im Zuge der Farbstoffsynthese selbst, ohne Zwischenisolierung des Farbstoffs, herzustellen.

Die Anwendungsform der wäßrigen Farbstoffpräparate ist insbesondere beim Färben oder Bedrucken von Papier bevorzugt. Die Herstellung einer stabilen, wäßrig konzentrierten Färbepräparation kann auf allgemeine Weise erfolgen, durch Lösen des Farbstoffs in Wasser gegebenenfalls unter Zugabe eines oder mehrerer Hilfsmittel, z.B. einer hydrotropen Verbindung oder eines Stabilisators.

Die wäßrigen Farbstoffpräparationen enthalten im allgemeinen etwa 0,5 bis 20 Gew.-% eines oder mehrerer Farbstoffe der Formel (I) und 80 bis 99,5 Gew.-% Wasser und/oder Lösungsmittel sowie gegebenenfalls weitere übliche Bestandteile.

Bevorzugte organische Lösungsmittel sind dabei Alkohole und deren Ether oder Ester, Carbonsäureamide, Harnstoffe, Sulfoxide und Sulfone, insbesondere solche mit Molekulargewichten < 200. Besonders geeignete Lösungsmittel sind beispielsweise: Methanol, Ethanol, Propanol; Ethylen-, Propylen-, Diethylen-, Thiodiethylen- und Dipropylen-glykol; Butandiol; β-Hydroxypropionitril, Pentamethylenglykol, Ethylenglykolmonoethyl- und propylether, Ethylendiglykolmonoethylether, Triethylenglykolmonobutylether, Butylpolyglykol, Formamid, Triethylenglykol, 1,5-Pentandiol, 1,3,6-Hexantriol, Essigsäure-2-hydroxyethylester, Glycerin, Glykolacetat, 1,2-Dihydroxypropan, 1-Methoxy-2-propanol, 2-Methoxy-1-propanol, N,N-Dimethylformamid, Pyrrolidon, N-Methyl-caprolactam, ε-Caprolactam, Butyrolacton, Harnstoff, Tetramethylharnstoff, 1,3-Dimethyl-2-imidazolidinon, N,N'-Dimethylolpropylenharnstoff, Dimethylsulfoxid, Dimethylsulfon, Sulfolan, Isopropanol, Polyethylenglykol.

Als weitere für wäßrige Farbstoffpräparationen, insbesondere für Drucktinten übliche Zusätze kommen solche ionischen oder nichtionischen Stoffe in Frage, mit denen die Viskosität und/oder Oberflächenspannung in die für die Anwendung erforderlichen Bereiche eingestellt werden kann, wie beispielsweise anionische, kationische oder neutrale Tenside wie Dispergiermittel und Viskositätsregulatoren. Die Funktion von Viskositätsregulatoren kann beispielsweise von den organischen Lösungsmitteln übernommen werden.

Die erfindungsgemäßen Farbstoffpräparationen können auch weitere Farbstoffe enthalten, die nicht der Formel I entsprechen. Beispielhaft kann Food Black 2 genannt werden.

Bevorzugt sind wäßrige Farbstoff-Präparationen, insbesondere Farbstoff-Lösungen, enthaltend
- 0,5 bis 20 Gew.-%, insbesondere 1 bis 15 Gew.-% eines oder mehrerer Farbstoffe, wobei mindestens einer der Formel I entspricht,
- 50 bis 99,5 Gew.-%, insbesondere 85 bis 99 Gew.-% Wasser,
- 0 bis 30 Gew.-%, insbesondere 0 bis 20 Gew.-% eines oder mehrere organische Lösungsmittel,
- 0 bis 30 Gew.-%, insbesondere 0 bis 10 Gew.-% die Viskosität und/oder die Oberflächenspannung beeinflussende Zusätze,
wobei sich die Summe der genannten Inhaltsstoffe auf 100 Gew.-% ergänzt.

Die wäßrigen Farbstoffpräparationen können durch Auflösen der Farbstoffsalze in Wasser oder aus den Kondensationslösungen, die gegebenenfalls einem Isomerenaustausch und/oder einer Entsalzung z.B. durch Druckpermeation unterworfen werden und/oder durch Zusatz eines oder mehrerer der obengenannten organischen Lösungsmitteln gegebenenfalls bei erhöhten Temperaturen (30 bis 100°C, insbesondere 30 bis 50°C) und unter Zusatz von anorganischen und organischen Basen hergestellt werden; gegebenenfalls können zusätzlich noch übliche ionische oder nichtionische Zusatzstoffe verwendet werden, z.B. solche, mit denen die Viskosität erniedrigt und/oder die Oberflächenspannung erhöht werden können.

Anstelle der Salze von (I) können auch die entsprechenden freien Säuren in Kombination mit mindestens äquimolaren Mengen der entsprechenden organischen oder organischen Basen eingesetzt werden.

Als anorganische Basen können z.B. Lithiumhydroxid, Natriumhydroxid, Kaliumhydroxid, Lithiumcarbonat, Natriumcarbonat, Kaliumcarbonat eingesetzt werden.

Als organische Basen können z.B. Ethanolamin, Diethanolamin, Triethanolamin, N-Methylethanolamin, N,N-Dimethylethanolamin, N-Methyldiethanolamin, 1-Amino-2-propanol, 2-Amino-1-propanol, Di-isopropanolamin, N-2-Hydroxyethyldiisopropanolamin, N,N,N-Tris-[2-[2'-hydroxyethoxy)-ethyl]-amin oder Natriummethylat, Lithiumethylat, Kalium-tert.-butylat dienen.

Die erfindungsgemäßen wäßrigen Farbstoffpräparationen eignen sich weiterhin zur Herstellung von Drucktinten, die insbesondere auch als Aufzeichnungsflüssigkeiten nach der Ink-Jet-Methode eingesetzt werden können.

Weiterer Gegenstand der Erfindung sind daher Drucktinten enthaltend mindestens einen Farbstoff (I) sowie deren Verwendung als Aufzeichnungsflüssigkeit für Ink-Jet-Aufzeichnungssysteme zur Erzeugung schwarzer Drucke.

Unter der Ink-Jet-Methode des erfindungsgemäßen Verfahrens wird ein Tintenstrahlaufzeichnungsverfahren verstanden, bei dem die Tintentropfen auf das Substrat gespritzt werden. Die feinen Tintentröpfchen können durch unterschiedliche Verfahren erzeugt werden. Bevorzugt werden sie nach den allgemein bekannten Thermal-Jet, Bubble Jet, Piezzo Jet oder Ventil-Ink-Jet-Verfahren erzeugt.

Bei der Verwendung der erfindungsgemäßen Farbstoffe in Form ihrer wäßrigen Präparationen, insbesondere ihrer Drucktinten als Aufzeichnungsflüssigkeit für Ink-Jet-Aufzeichnu'ngssysteme ergeben sich folgende Vorteile: Die physikalischen Eigenschaften, wie Viskosität, Oberflächenspannung und dergleichen, liegen in den geeigneten Bereichen; die Aufzeichnungsflüssigkeit verursacht keine Verstopfungen in feinen Abgabeöffnungen von Tintenstrahl-Aufzeichnungsvorrichtungen; sie liefert Bilder von hoher Dichte; bei der Lagerung kommt es in der Aufzeichnungsflüssigkeit nicht zu einer Veränderung von physikalischen Eigenschaften und zur Ablagerung von festen Bestandteilen; die Aufzeichnungsflüssigkeit eignet sich zur Aufzeichnung auf verschiedenen Aufzeichnungsmedien ohne Beschränkungen hinsichtlich der Art der Aufzeichnungsmedien; schließlich fixiert die Aufzeichnungsflüssigkeit rasch und ergibt Bilder mit ausgezeichneter Wasserfestigkeit, Lichtechtheit, Abriebbeständigkeit und Auflösung.

Die folgenden Herstellungsbeispiele sollen die vorliegende Erfindung erläutern ohne sie jedoch darauf zu beschränken. In den Beispielen bedeuten Teile stets Gewichtsteile, falls nichts anderes angegeben ist.

Die mit den erfindungsgemäßen Farbstoffen erhaltenen Färbungen bzw. Drucke, insbesondere die Drucke, die nach der Ink-Jet-Methode auf Papier erhalten wurden, weisen hervorragende Naßechtheiten auf.

### Beispiele

### Beispiel 1

0,55 Mol Cyanurchlorid wurden in 500 ml Aceton gelöst und bei 0 bis 5°C innerhalb von 20 Minuten mit 0,5 Mol 1-Amino-3-nitrobenzol portionsweise versetzt und 30 Minuten nachgerührt. Danach tropfte man 100 ml 20%ige Sodalösung dazu, rührte erneut 1 Stunde bei 0°C nach und trug dann auf 1 l Eiswasser aus. Nach Absaugen des Niederschlages wurde gut abgepreßt und mit 0,5 l Eiswasser gewaschen. Der Rückstand wurde in einen Überschuß von 2-Hydroxyethylpiperazin eingetragen, wobei die Temperatur auf 105°C anstieg. Es wurde 1 Stunde bei 100°C gerührt, über Nacht abgekühlt, in Eiswasser ausgetragen, abgesaugt, mit Eiswasser gewaschen und gut abgepreßt. Der Rückstand wurde in 750 ml Ethanol gelöst und mit Raney-Nickel bei 50°C im Autoklaven bis zur Druckkonstanz hydriert. Nach dem Abfiltrieren des Katalysators wurde eingeengt, der Rückstand mit Wasser aufgenommen und das Produkt mit Salzsäure in Lösung gebracht. Man erhält die Kupplungskomponente der Formel 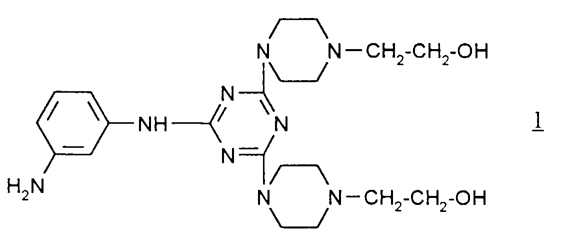 Ersetzte man in Beispiel 1 das 2-Hydroxyethylpiperazin durch die folgenden Amine der Tabelle 1, erhielt man weitere wichtige Kupplungskomponenten der Formel

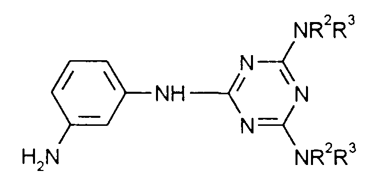

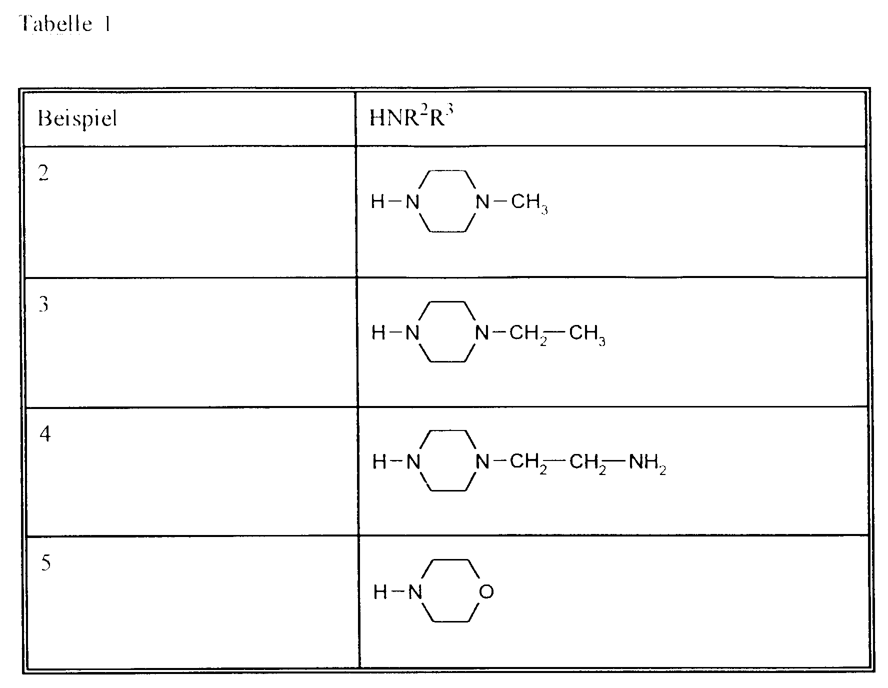

### Beispiel 6

0,07 Mol der Verbindung der Formel 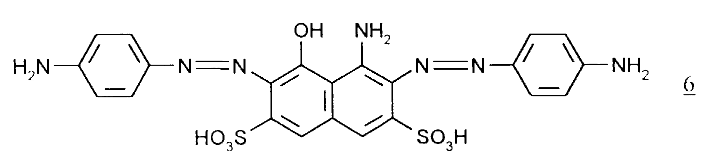 wurden in 250 ml Wasser angerührt, mit Soda auf pH 8 gestellt, mit NaNO₂ - Lösung versetzt und zu einer Lösung aus 120 ml HCl und 250 g Eis getropft und diazotiert. Nachdem 1 Stunde mit Nitritüberschuß nachgerührt wurde, wurde dieser mit Amidosulfonsäure zerstört. Zu dieser Diazotierungs-Reaktionsmischung wurden 0,15 Mol der Kupplungskomponente der Formel (I) gegeben und der pH-Wert mit festem Natriumacetat auf pH 4,5 gestellt und 2 Stunden gerührt. Dann wurde mit Sodalösung auf pH 8 gestellt, über Nacht gerührt, abgesaugt, mit Wasser gewaschen und getrocknet. Man erhielt den Farbstoff der Formel 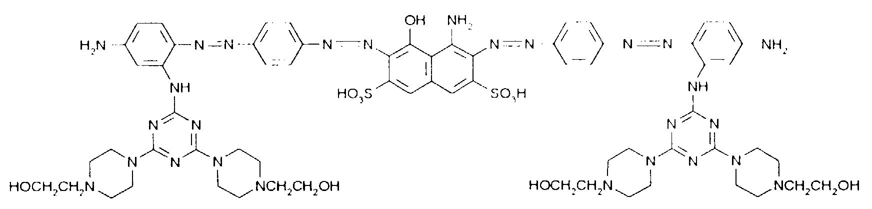 der Papier in grünschwarzen Tönen anfärbt.

Ersetzte man in Beispiel 6 die Kupplungskomponente des Beispiels 1 durch eine Kupplungskomponente der Tabelle 1, erhielt man weitere wertvolle Farbstoffe der Formel 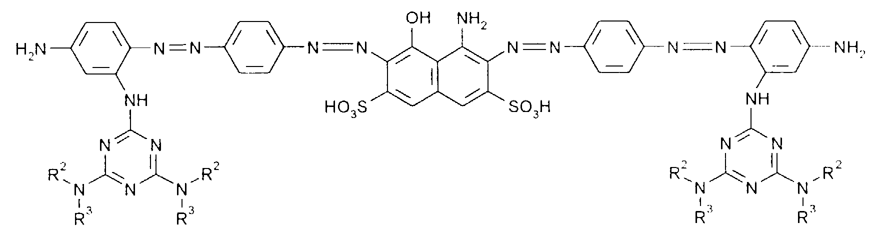 die in Tabelle 2 aufgeführt sind.

**Tabelle 2**

| Beispiel | Kupplungskomponente aus Beispiel |
|---|---|
| 7 | 2 |
| 8 | 3 |
| 9 | 4 |
| 10 | 5 |

### Beispiele 11 bis 15

Ersetzte man in den Beispielen 6 bis 10 die Verbindung der Formel 6 durch die Verbindung der Formel 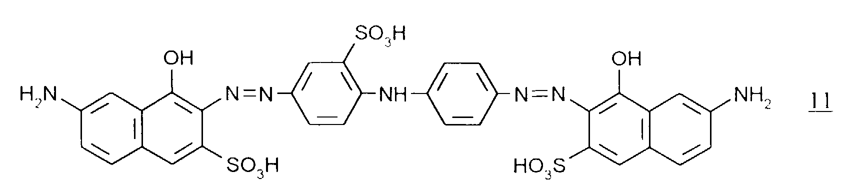 erhielt man Farbstoffe der Formel 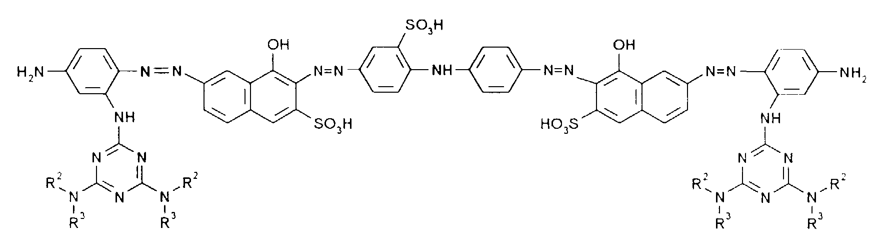 die in der Tabelle 3 angegeben sind, und die Papier in blaustichigen Schwarztönen färbten.

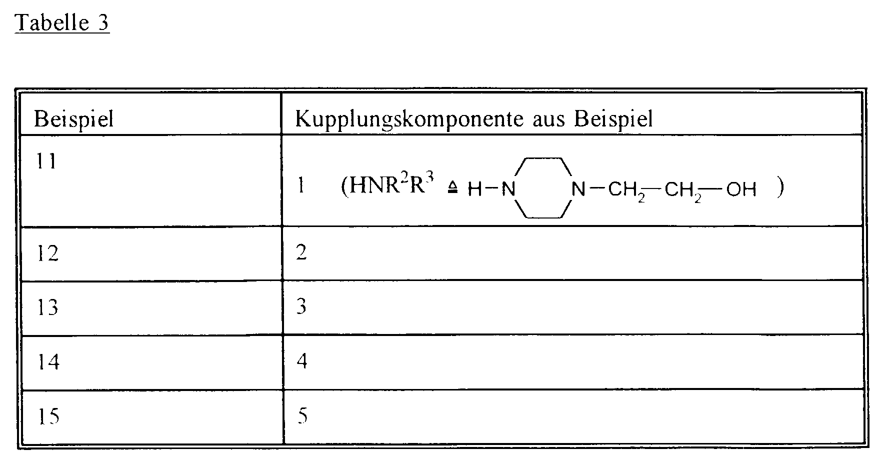

### Beispiel 16

0,29 Mol Metaminsäure wurden in Wasser suspendiert, mit NaOH gelöst und zu 0,3 Mol Cyanurchlorid in Eiswasser eingestürzt. Der pH-Wert wurde mit Sodalösung hei 4 bis 4,5 gehalten und 1 Stunde bei 15°C nachgerührt. Danach wurde das Kondensationsprodukt abgetrennt und in einen Überschuß an N-Ethylpiperazin/Eiswasser eingetragen. Die Lösung erwärmte sich auf 45°C und wurde dann weiter auf 95°C aufgeheizt und 1 Stunde gerührt. Nach dem Abkühlen fiel das Produkt der Formel 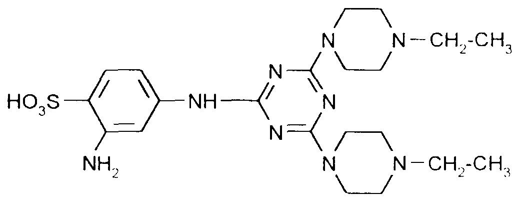 aus, wurde abgesaugt und getrocknet.

Ersetzte man in Beispiel 16 das N-Ethylpiperazin durch die Amine der Tabelle 4, erhielt man weitere wichtige Kupplungskomponenten der Formel

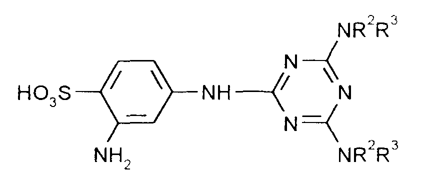

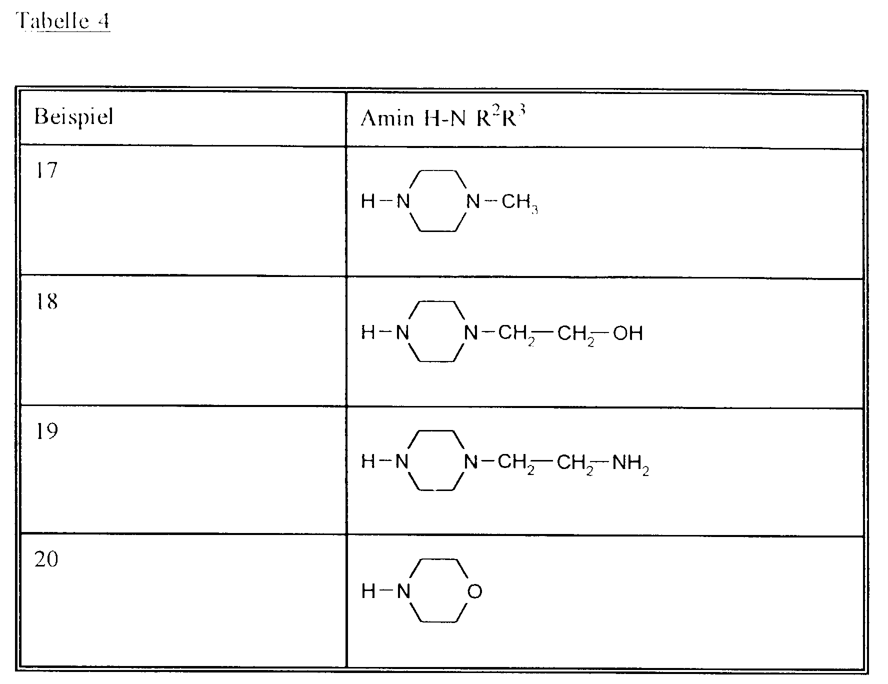

### Beispiele 21 bis 25

Ersetzte man in Beispiel 6 die Kupplungskomponente der Formel 1 durch die Kupplungskomponente der Beispiele 16 bis 20, erhielt man die Farbstoffe der Formel 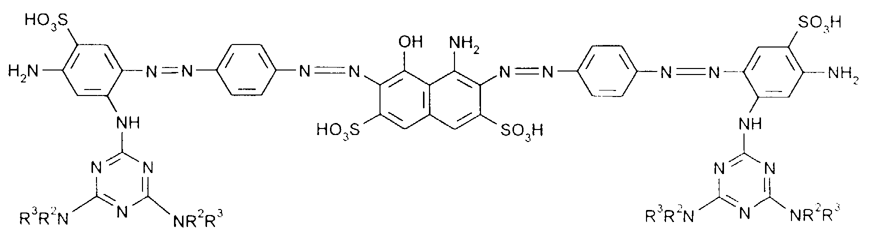 die in der Tabelle 5 angegeben sind, und die Papier ebenfalls in schwarzen Tönen färben.

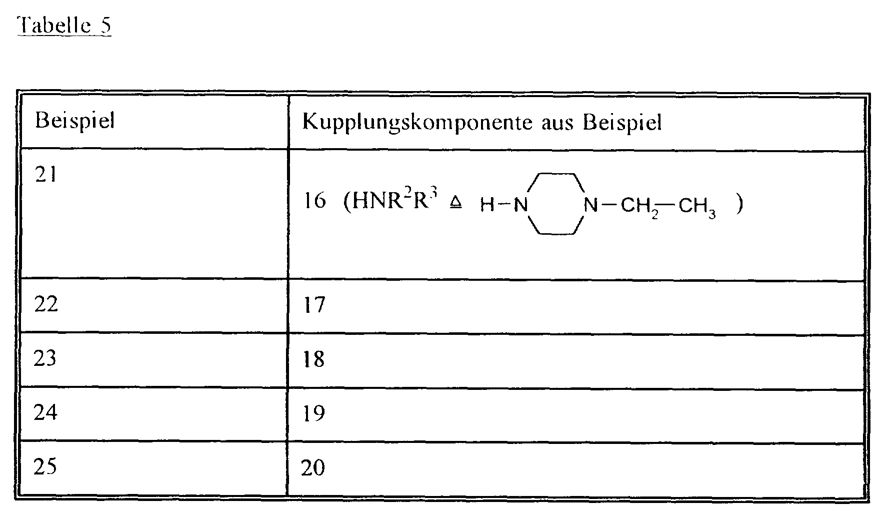

### Beispiele 26 bis 32

Ersetzte man in den Beispielen 21 bis 25 die Tetrazokomponente durch die des Beispiels 11, erhielt man die Verbindungen der Formel 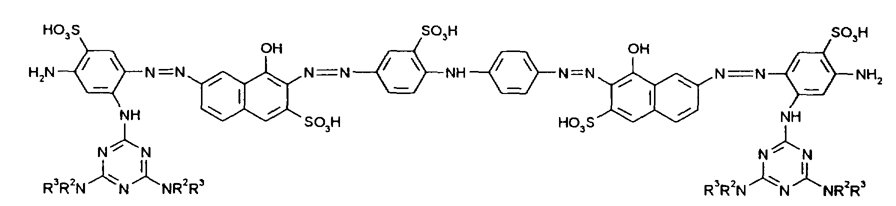 die in der Tabelle 6 angegeben sind.

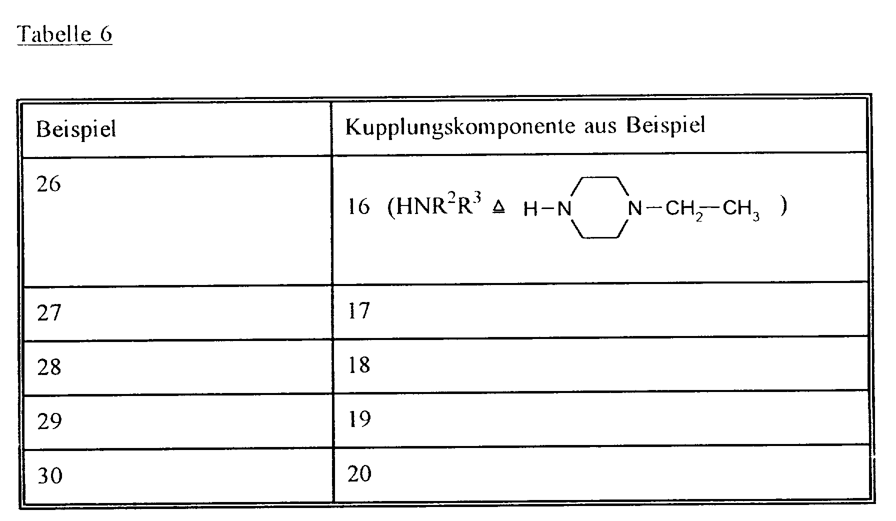

### Beispiele 31 bis 40

Ersetzte man in den Beispielen 6 bis 10 wand 21 bis 25 die Tetrazokomponente der Formel 6 durch die Verbindung der Formel 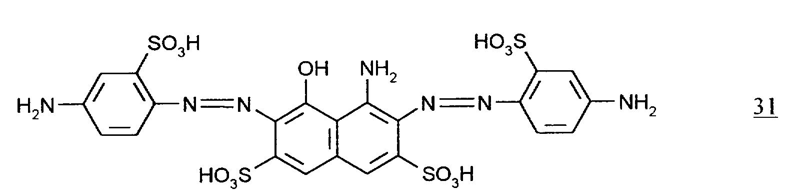 die durch saure Kupplung von Oxalylparaminsäure in Gegenwart von Harnstoff, anschließender alkalischer Kupplung von Oxalylparaminsäure und alkalischer Verseifung der Oxalylgruppen hergestellt wird, erhielt man Farbstoffe der Formel 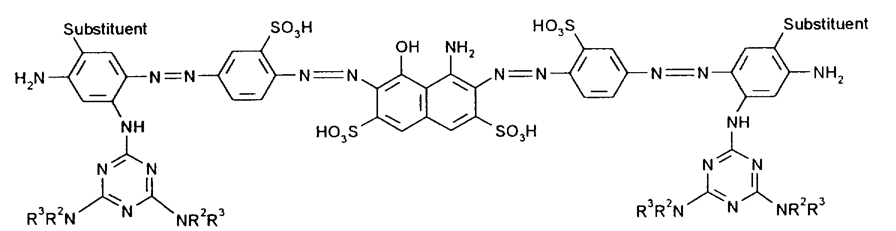 die in der Tabelle 7 angegeben sind, und die Papier in blaustichigen Schwarztönen färben.

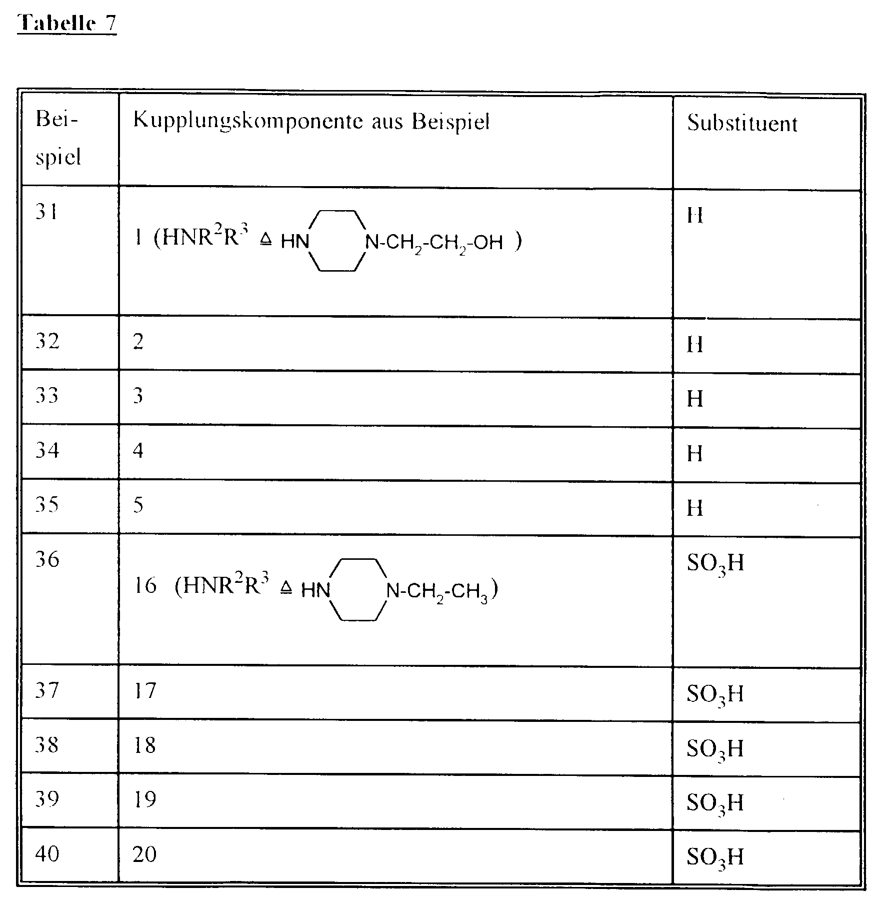

### Beispiele 41 bis 50

Ersetzte man in den Beispielen 31 bis 40 die Tetrazokomponente 31 durch die Verbindung der Formel 41 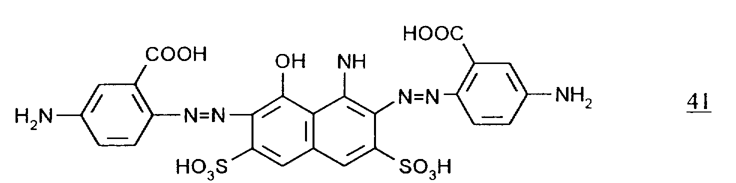 die durch saure und alkalische Kupplung von 5-Nitro-2-aminobenzoesäure mit H-Säure und anschließender NaHS-Reduktion hergestellt wurde, erhielt man Farbstoffe der Formel 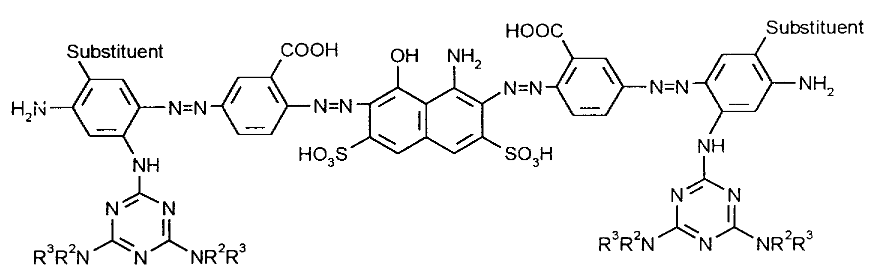 die in der Tabelle 8 angegeben sind, und die Papier in blaustichigen Schwarztönen färben.

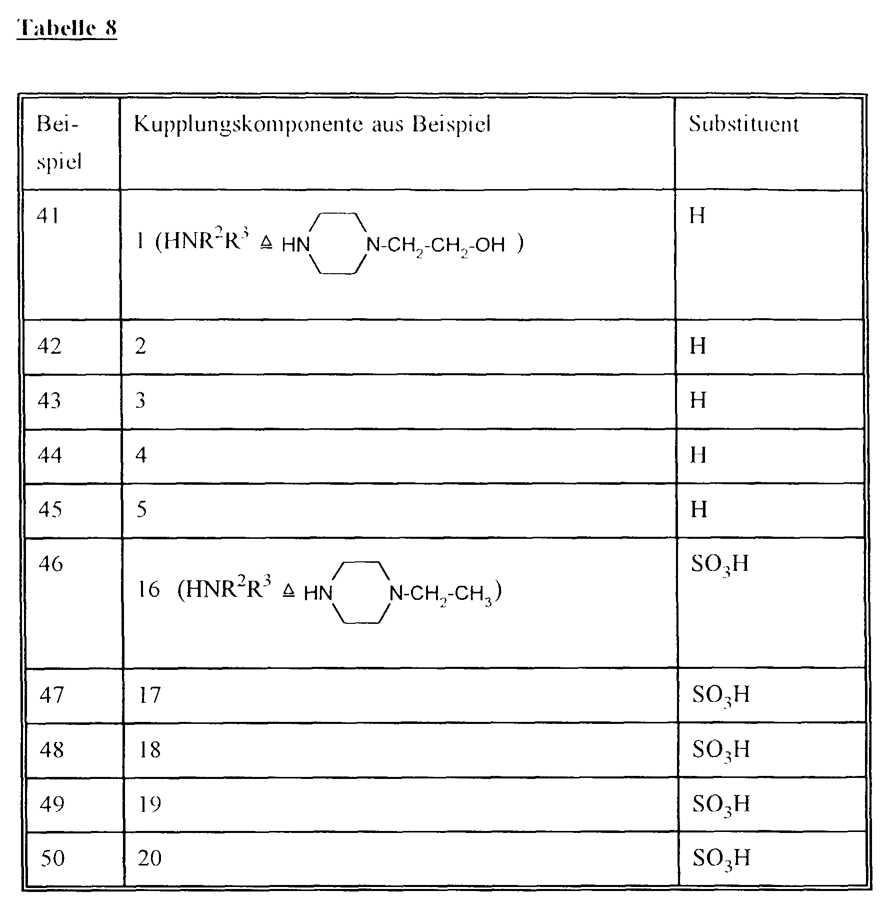

### Beispiel 51 bis 60

Ersetzte man in den Beispielen 41 bis 50 die Tetrazokomponente 41 durch die Verbindung der Formel 51 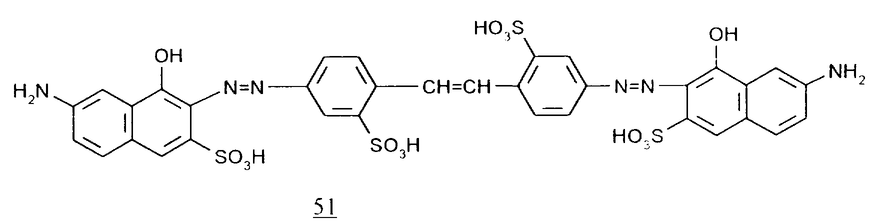 erhielt man Farbstoffe der Formel 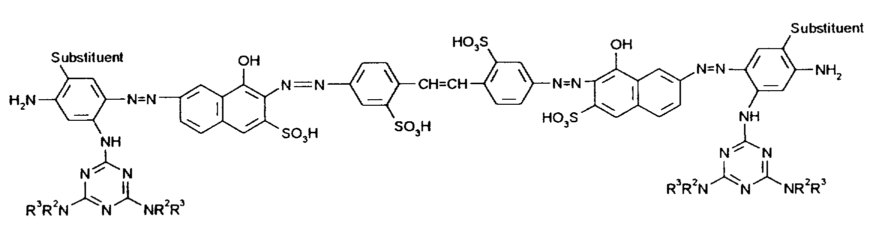 die in der Tabelle 9 angegeben sind, und die Papier in grünstichig schwarzen Tönen färbten.

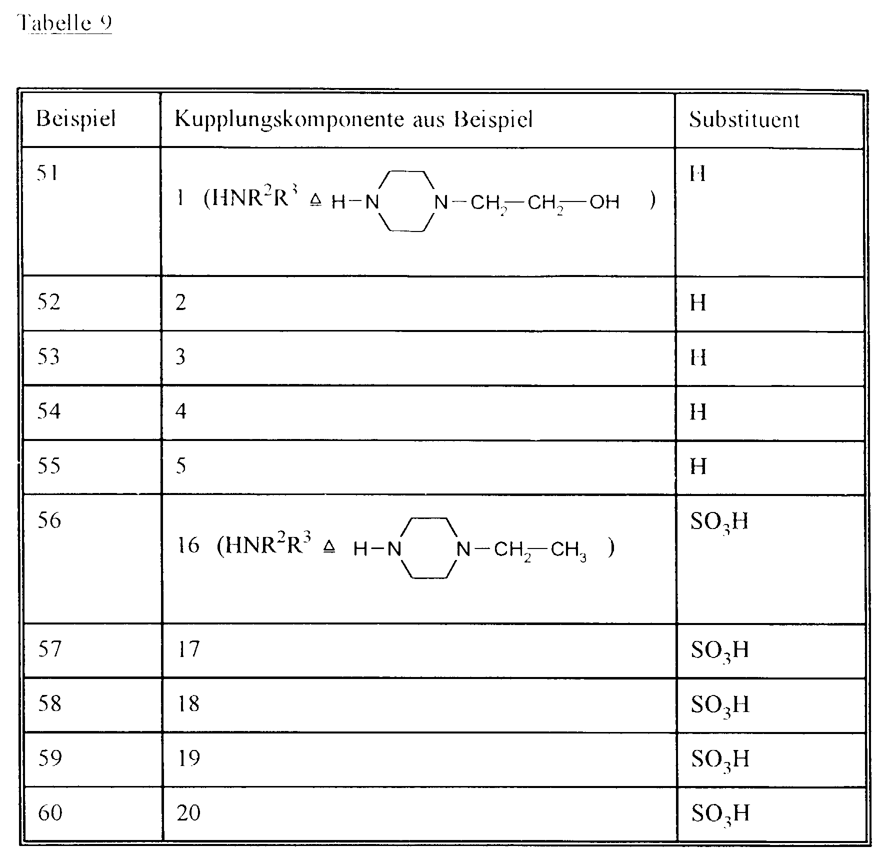

## Patentansprüche

1. Verbindungen der Formel (I) 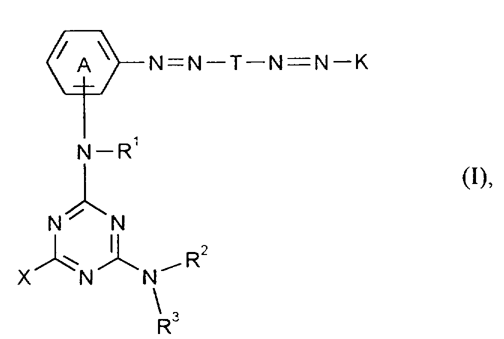 worin
T für den Rest einer Tetrazokomponente steht,
R¹ Wasserstoff oder unsubstituiertes oder substituiertes C₁-C₄-Alkyl bedeutet,
R² und R³ zusammen mit dem N-Atom, an das sie gebunden sind, einen gesättigten oder ungesättigten 3- bis 8-, insbesondere 5- oder 6-gliedrigen Ring bilden, der entweder keine weiteren oder 1 bis 2 weitere Heteroatome aus der Gruppe O, S, SO, SO₂, NR, mit R = H oder unsubstituiertes oder substituiertes C₁-C₆-Alkyl, enthält und unsubstituiert oder durch C₁-C₄-Alkyl, C₁-C₄-Alkoxy, C₁-C₄-Hydroalkyl oder C₁-C₄-Aminoalkyl substituiert ist,
X für NR²R³ steht, wobei R² und R³ die obengenannte Bedeutung besitzen, und der Ring A gegebenenfalls weitere Substituenten trägt,
K einen Rest der Formel (II) bedeutet, 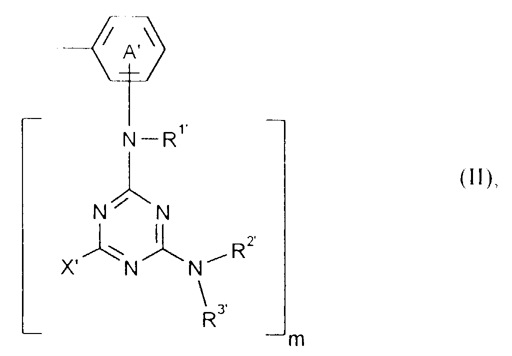
worin
R^{1'} Wasserstoff oder unsubstituiertes oder substituiertes C₁-C₄-Alkyl bedeutet,
R^{2'} und R^{3'} zusammen mit dem N-Atom, an das sie gebunden sind, einen gesättigten oder ungesättigten 3- bis 8-, insbesondere 5- oder 6-gliedrigen Ring bilden, der entweder keine weiteren oder 1 bis 2 weitere Heteroatome aus der Gruppe O, S, SO, SO₂, NR, mit R = H oder unsubstituiertes oder substituiertes C₁-C₄-Alkyl, enthält und unsubstituiert oder durch C₁-C₄-Alkyl, C₁-C₄-Alkoxy, C₁-C₄-Hydroalkyl oder C₁-C₄-Aminoalkyl substituiert ist,
X' für NR^{2'}R^{3'} steht, wobei R^{2'} und R^{3'} die oben genannte Bedeutung besitzen, und der Ring A' gegebenenfalls weitere Substituenten trägt und
m für 1 steht.

2. Verbindungen nach Anspruch 1, worin m = 1, A' = A, R^{1'}= R¹, R^{2'}= R², R^{3'}= R³ und X' = X bedeutet.

3. Verbindungen nach Anspruch 1, worin der Rest der Tetrazokomponente T der Formel (III) 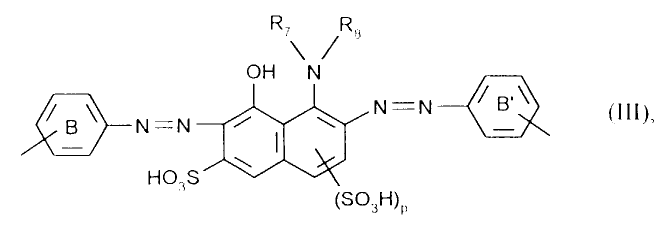 entspricht, worin
R⁷ und R⁸ unabhängig voneinander Wasserstoff oder unsubstituiertes oder substituiertes C₁-C₄-Alkyl bedeuten,
die Ringe B und B' unabhängig voneinander entweder keine weiteren oder weitere Substituenten tragen und p 0 oder 1 bedeutet.

4. Verbindungen nach Anspruch 1, worin der Rest der Tetrazokomponente T der Formel (IV) 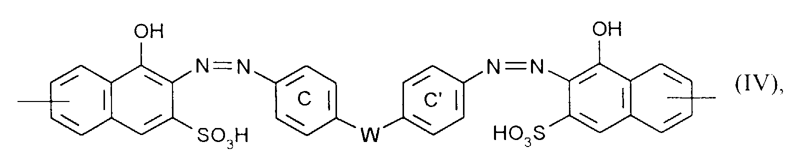 enspricht, worin
die Ringe C und C' unabhängig voneinander entweder keine weiteren oder weitere Substituenten tragen und
W für ein Brückenglied steht.

5. Verbindungen nach Anspruch 1, worin 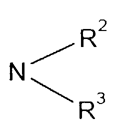 einen Rest der Formel 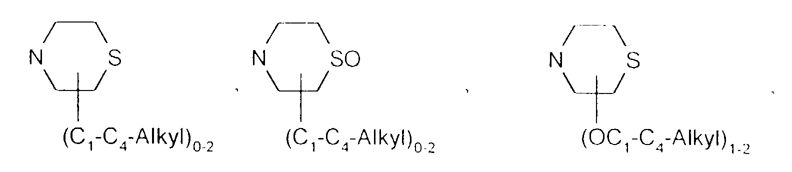 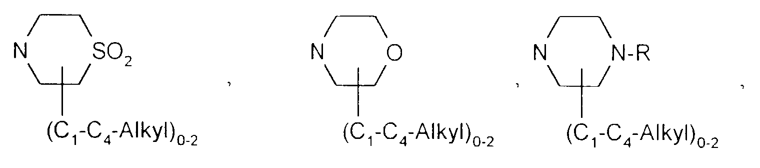 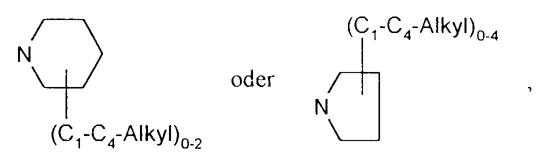 worin
R die in Anspruch 1 angegebene breiteste Bedeutung hat.

6. Verbindungen nach Anspruch 1, die der Formel (VI) entsprechen

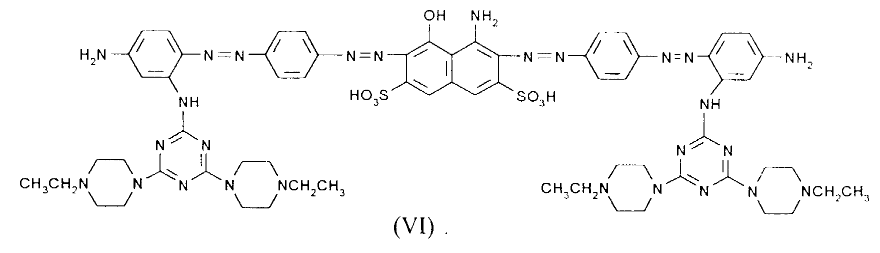

7. Verfahren zur Herstellung von Verbindungen gemäß Anspruch 1, gekennzeichnet durch Tetrazotierung der Diamine der Formel (VII)
H₂N - T - NH₂ (VII)
und Kupplung auf die Kupplungskomponenten der Formel 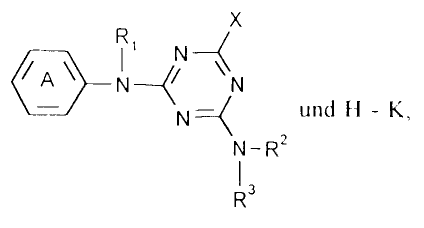 worin
R¹, R², R³, A, X, T und K die in Anspruch 1 angegebene Bedeutung haben.

8. Wäßrige Farbstoff-Präparationen, insbesondere Farbstoff-Lösungen, enthaltend
- 0,5 bis 20 Gew.-% eines oder mehrerer Farbstoffe, wobei mindestens einer davon der Verbindung des Anspruchs 1 entspricht,
- 50 bis 99,5 Gew.-% Wasser,
- 0 bis 30 Gew.-% eines oder mehrerer organische Lösungsmittel,
- 0 bis 30 Gew.-% die Viskosität und/oder der Oberflächenspannungsbeeinflussende Zusätze, wobei sich die Summe der genannten Inhaltsstoffe auf 100 % ergänzt.

9. Drucktinten enthaltend mindestens einen Farbstoff, der einer Verbindung gemäß Anspruch 1 entspricht.

10. Verwendung der Verbindungen des Anspruchs 1 zum Farben und Bedrucken von hydroxy- und/oder amidgruppenhaltigen Substraten, insbesondere zum Bedrucken von Papier nach der Ink-Jet-Methode.

## Claims

1. Compounds of the formula (I) 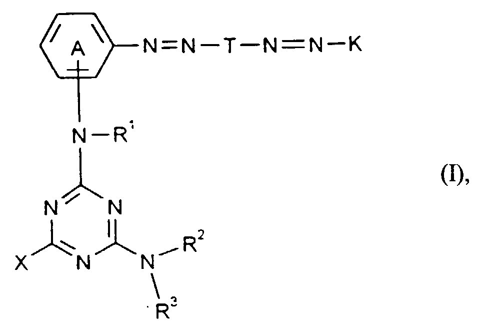 in which
T represents the radical of a tetrazo component,
R¹ denotes hydrogen or substituted or unsubstituted C₁-C₄-alkyl,
R² and R³ together with the N atom to which they are bonded form a saturated or unsaturated 3- to 8-membered, in particular 5- or 6-membered, ring which either does not contain any further heteroatoms or contains 1 to 2 further heteroatoms from the group consisting of O, S, SO, SO₂, NR, where R is H or substituted or unsubstituted C₁-C₆-alkyl, and is unsubstituted or substituted by C₁-C₄-alkyl, C₁-C₄-alkoxy, C₁-C₄-hydroxyalkyl or C₁-C₄-aminoalkyl,
X represents NR²R³ where R² and R³ have the meaning given above, and ring A does, if desired, carry further substituents,
K denotes a radical of the formula (II) 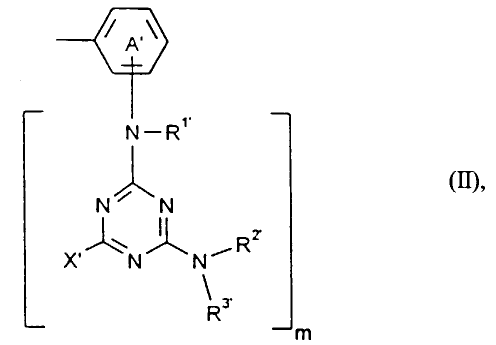
in which
R^{1'} denotes hydrogen or substituted or unsubstituted C₁-C₄-alkyl,
R^{2'} and R^{3'} together with the N atom to which they are bonded form a saturated or unsaturated 3- to 8-membered, in particular 5- or 6-membered, ring which either does not contain any further heteroatoms or contains 1 to 2 further heteroatoms from the group consisting of O, S, SO, SO₂, NR, where R is H or substituted or unsubstituted C₁-C₄-alkyl and is unsubstituted or substituted by C₁-C₄-alkyl, C₁-C₄-alkoxy, C₁-C₄-hydroxyalkyl or C₁-C₄-aminoalkyl,
X' represents NR^{2'}R^{3'} where R^{2'} and R^{3'} have the meaning given above, and ring A' does, if desired, carry further substituents and
m represents 1.

2. Compounds according to Claim 1, in which m is 1, A' is A, R^{1'} is R¹, R^{2'} is R², R^{3'} is R³ and X' is X.

3. Compounds according to Claim 1, in which the radical of the tetrazo component T has the formula (III) 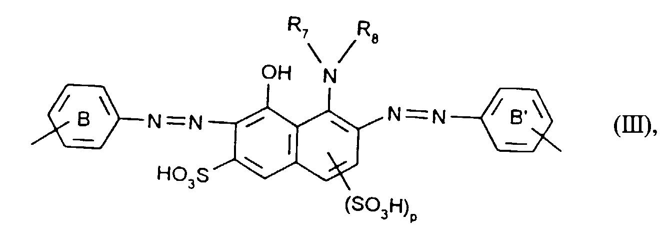 in which
R⁷ and R⁸, independently of one another, denote hydrogen or substituted or unsubstituted C₁-C₄-alkyl,
rings B and B', independently of one another, either do not carry any further substituents or carry further substituents and p denotes 0 or 1.

4. Compounds according to Claim 1, in which the radical of the tetrazo component T has the formula (IV) 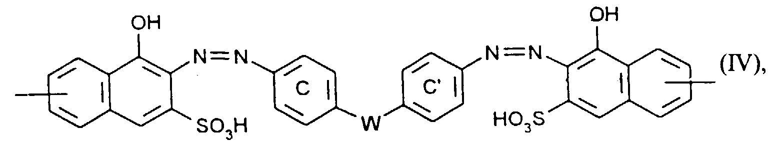 in which
rings C and C', independently of one another, either do not carry any further substituents or carry further substituents and
W represents a bridging member.

5. Compounds according to Claim 1, in which 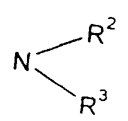 denotes a radical of the formula 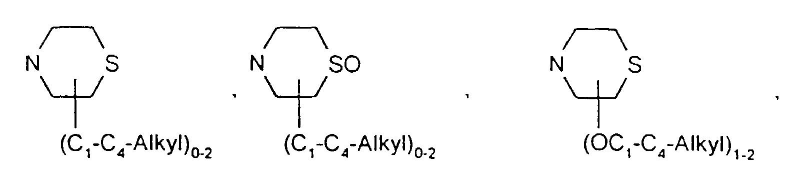 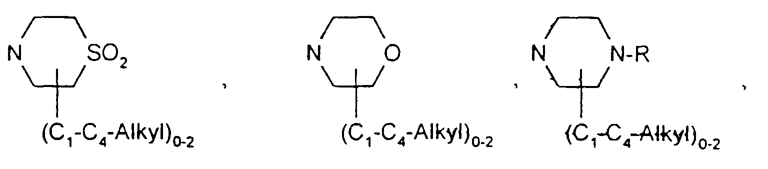 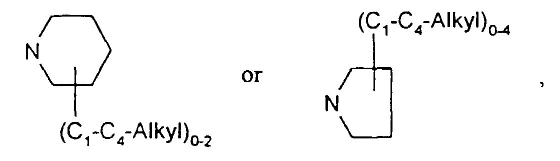 in which
R has the broadest meaning given in Claim 1.

6. Compounds according to Claim 1, having the formula (VI)

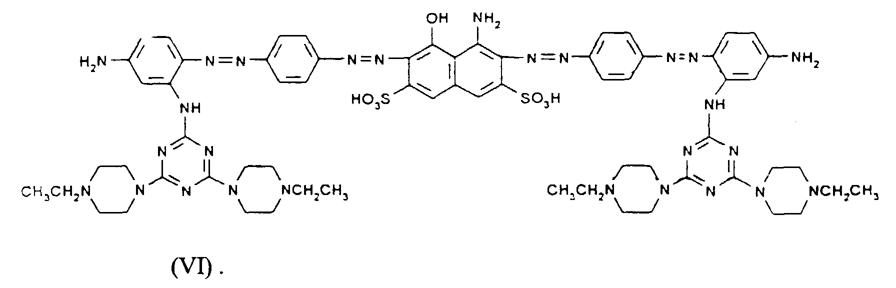

7. Process for preparing compounds according to Claim 1, characterized by tetrazotization of the diamines of the formula (VII)
H ₂N - T - NH₂ (VII)
and coupling of the resulting tetrazonium salts onto coupling components of the formula 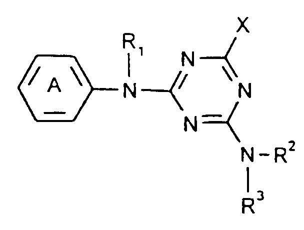 and H - K,
in which
R¹, R², R³, A, X, T and K have the meaning given in Claim 1.

8. Aqueous dyestuff preparations, in particular dyestuff solutions, containing
- 0.5 to 20% by weight of one or more dyestuffs, at least one of which is a compound according to Claim 1,
- 50 to 99.5% by weight of water,
- 0 to 30% by weight of one or more organic solvents,
- 0 to 30% by weight of additives affecting the viscosity and/or surface tension, the sum of the ingredients mentioned adding up to 100% by weight.

9. Printing inks, containing at least one dyestuff which is a compound according to Claim 1.

10. Use of the compounds of Claim 1 for dyeing and printing hydroxyl- and/or amido-containing substrates, in particular for printing paper by the ink-jet method.

## Revendications

1. Composés de formule (I) 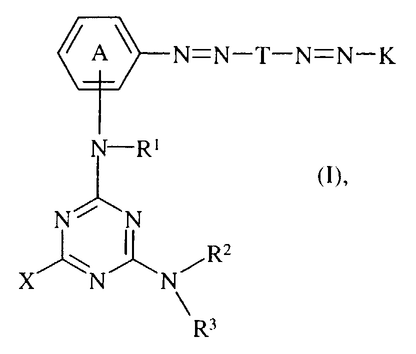 dans laquelle
T représente le radical d'un composant tétrazoïque,
R¹ représente l'hydrogène ou un groupe alkyle en C₁-C₄ éventuellement substitué,
R² et R³ représentent ensemble et avec l'atome d'azote auquel ils sont reliés un cycle saturé ou insaturé de 3 à 8, plus spécialement de 5 ou 6 chaînons, qui ne contient par d'autre hétéroatome ou qui contient 1 ou 2 autres hétéroatomes choisis parmi O, S, SO, SO₂, NR avec R = H ou alkyle en C₁-C₆ éventuellement substitué, et qui est non substitué ou substitué par des groupes alkyle en C₁-C₄, alcoxy en C₁-C₄, hydroxyalkyle en C₁-C₄ ou aminoalkyle en C₁-C₄,
X représente NR²R³ dans lequel R² et R³ ont les significations indiquées ci-dessus, et le cycle A porte éventuellement d'autres substituants,
K représente un radical de formule (II) 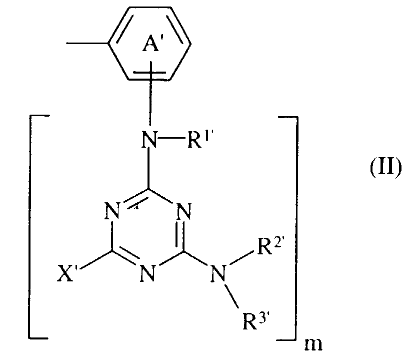
dans laquelle
R^{1'} représente l'hydrogène ou un groupe alkyle en C₁-C₄ éventuellement substitué,
R^{2'}et R^{3'} représentent ensemble et avec l'atome d'azote auquel ils sont reliés un cycle saturé ou insaturé de 3 à 8, plus spécialement de 5 ou 6 chaînons, qui ne contient par d'autre hétéroatome ou qui contient 1 ou 2 autres hétéroatomes choisis parmi O, S, SO, SO₂, NR avec R = H ou alkyle en C₁-C₄ éventuellement substitué, et qui est non substitué ou substitué par des groupes alkyle en C₁-C₄, alcoxy en C₁-C₄, hydroxyalkyle en C₁-C₄ ou aminoalkyle en C₁-C₄,
X' représente NR^{2'}R^{3'} dans lequel R^{2'} et R^{3'} ont les significations indiquées ci-dessus, et le cycle A' peut porter éventuellement d'autres substituants, et
m est égal à 1.

2. Composés selon la revendication 1, pour lesquels m = 1, A' = A, R^{1'} = R¹, R^{2'} = R², R^{3'} = R³ et X'= X.

3. Composés selon la revendication 1, pour lesquels le radical du composé tétrazoïque T répond à la formule (III) 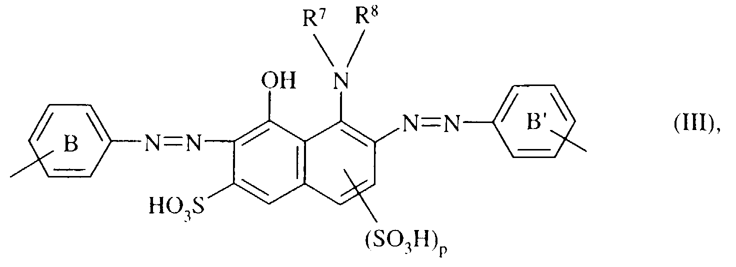 dans laquelle
R⁷ et R⁸ représentent chacun, indépendamment l'un de l'autre, l'hydrogène ou un groupe alkyle en C₁-C₄ éventuellement substitué,
les cycles B et B', indépendamment l'un de l'autre, ne portent pas d'autres substituants ou portent d'autres substituants, et p est égal à 0 ou 1.

4. Composés selon la revendication 1, pour lesquels le radical du composé tétrazoïque T répond à la formule (IV) 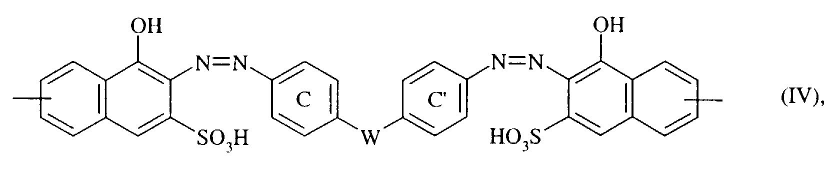 dans laquelle
les cycles C et C', indépendamment l'un de l'autre, ne portent pas d'autres substituants ou portent d'autres substituants et
W représente un pont.

5. Composés selon la revendication 1, pour lesquels 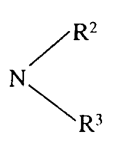 représente un groupe de formule 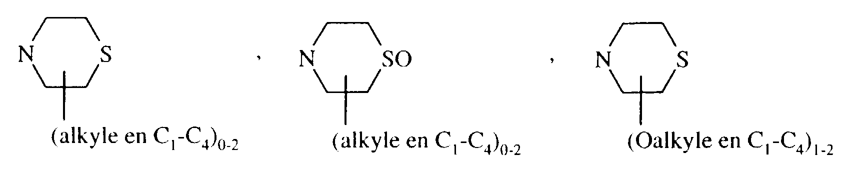 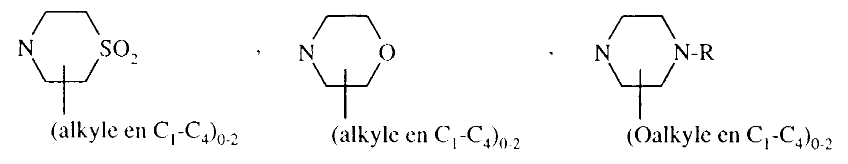 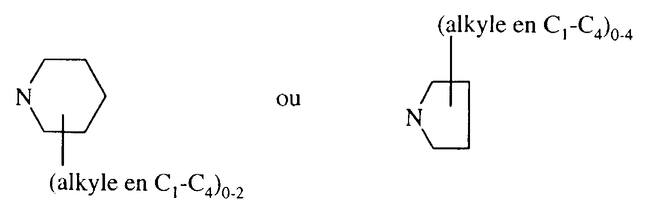 dans lesquelles
R a les significations les plus larges de la revendication 1.

6. Composés selon la revendication 1, répondant à la formule particulière (VI)

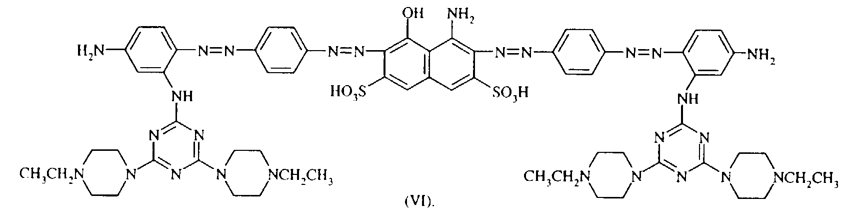

7. Procédé de préparation des composés de la revendication 1, caractérisé en ce que l'on tétrazote les diamines de formule (VII)
H₂N- T - NH₂ (VII)
et on copule sur les copulants de formules 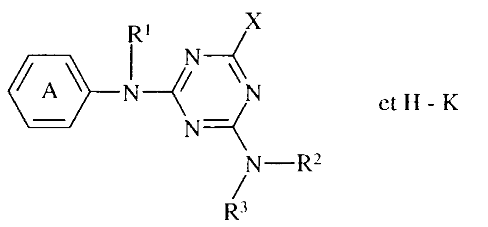 dans lesquelles
R¹, R², R³, A , X, T et K ont les significations indiquées dans la revendication 1.

8. Compositions aqueuses de colorants, en particulier solutions de colorants, contenant
- 0,5 à 20 % en poids d'un ou plusieurs colorants dont au moins un est un composé selon la revendication 1,
- 50 à 99,5 % en poids d'eau,
- 0 à 30 % en poids d'un ou plusieurs solvants organiques,
- 0 à 30 % en poids d'additifs agissant sur la viscosité et/ou sur la tension superficielle,
la somme des teneurs indiquées représentant 100 %.

9. Encres d'impression contenant au moins un colorant qui consiste en un composé selon la revendication 1.

10. Utilisation des composés de la revendication 1 pour la teinture et l'impression de supports contenant des groupes hydroxy et/ou amide, en particulier pour l'impression du papier par la technique à jet d'encre.
